Europäisches Patentamt

European Patent Office

Office européen des brevets

(11) Publication number: **0 222 069**
A2

## (12) EUROPEAN PATENT APPLICATION

(21) Application number: **86110669.8**

(22) Date of filing: **01.08.86**

(51) Int. Cl.⁴: **B 01 J 8/04**
**C 01 C 1/04, C 07 C 29/15**

(30) Priority: **13.09.85 CH 3949/85**

(43) Date of publication of application:
**20.05.87 Bulletin 87/21**

(84) Designated Contracting States:
**DE FR GB IT NL**

(71) Applicant: **AMMONIA CASALE S.A.**
**Via della Posta 4**
**CH-6900 Lugano(CH)**

(71) Applicant: **Zardi, Umberto**
**Via Lucino 57**
**CH-6932 Breganzona (Ti)(CH)**

(72) Inventor: **Zardi, Umberto**
**Via Lucino 57**
**CH-6900 Breganzona(CH)**

(74) Representative: **Incollingo, Italo**
**AMMONIA CASALE S.A. Via della Posta 4**
**CH-6900 Lugano(CH)**

(54) **Converter for heterogeneous synthesis more particularly for ammonia, methanol and higher alcohols.**

(57) Converters for heterogeneous synthesis and more particularly for ammonia, methanol and higher alcohols catalytic synthesis, which consist of an internal cartridge involving a non-perforated external wall, forming an interspace with the shell internal wall, of n catalytic beds, each formed by granular catalyst arranged between closed bottom and a top open surface, or between bottom and cover both closed, and two cylindrical, concentric, perforated walls, of means for adduction of the reaction gases, of means for extraction of reacted gases, of heat exchangers lined up axially at the center of the catalytic beds, which consist of tube-bundles, and of means for adduction to said tube-bundles of feed gas for heat exchange, are now characterized by:

- n adduction means of feed gas for heat exchange, n-1 of which centrally flow through each catalytic bed,
- means to lead the reaction gas flowing from top to bottom in the interspace between shell and cartridge, by making it pass externally to the tube-bundle of at least one lower heat exchanger, which is internally passed by the reacted gases directed to the converter outlet and by making said reaction gas pass internally to the tube-bundles of the other heat exchangers on its way back to the converter top and
- means to mix at the top of each exchanger said reaction gas and said exchange gases.

SPECIFICATION

-----------------

The invention refers to converters for heterogeneous synthesis and more particularly for catalytic synthesis of ammonia, methanol and higher alcohols, which consist of an external wall forming an interspace with the shell internal wall, of n catalytic beds formed by granular catalyst set between the closed bottom or closed bottom and cover, and by two perforated, cylindrical, concentric walls; of means of adduction for reaction gas; of means for extraction of reacted gases; of heat exchangers lined up axially at the center of the catalytic beds and comprising tube bundles and of means for adduction of fresh exchange gas to said tube-bundles.

Converters of that kind are described in literature in the most diversified forms of realization: particularly in recent patents and patent applications of the Applicant, very advantageous converters are being described in which, each layer or catalytic bed is passed through by reaction gases in a zone with mainly axial flow and in another zone with mainly radial flow, the zone with mainly axial flow acting at the same time as sealing cap between the catalytic layers.

Preferably, the internal cartridge is advantageously obtained by simply assembling modular cartridges, each one formed (proceeding from the outside to the inside) by a lateral, cylindrical, non-perforated bottom that can be the extension of the non-perforated external wall, by a first cylindrical wall, perforated on an axial major extension and by a cylindrical, internal wall only partly perforated, i.e., having at least one small, axial, non-perforated extension:

the catalytic bed is put on said bottom and is delimited by said lateral, partly perforated walls. Each layer or catalytic bed is open at the top, where the zone with a mainly axial flow is passed through by a minor portion of gas going through the remaining major zone of the catalytic bed with mainly radial flow.

Although it is well known the importance of realizing converters with more catalytic beds with temperature control of the gas obtained by means of direct injection of fresh gas at a lower temperature (quench reactors), by means of direct interchange between feeding gas at a lower temperature and hot gas coming out from each single bed, advantageously carried out by means of exchangers allowing higher conversion efficiency, in the state of art, particularly in the Applicant's previous patents, converters with a variety of catalytic beds are being described, but with a temperature control between each bed realized by means of direct injection of fresh gas, which, as above mentioned, reduces the efficiency, or with two catalytic beds and temperature control between the beds by means of a single interchange on an exchanger between fresh gas and gas coming out of the first bed.

Exceptionally, converters are described, which have three catalytic beds and two interchanges by means of exchanger between the beds, but with extremely onerous and complicated embodiments.

Carrying forward its researches, the Applicant has now found that it is possible to improve the efficiency and the conditions of the synthesis reactions with a critical choice of high efficiency cartridges, with more catalytic beds (at least three) for synthesis converters, which cartridges can be conveniently realized according to the present invention with:

I   -   A flow (A) from the top down to the bottom of fresh reaction gas (GR) in the interspace (10) between shell (M) and external wall of the cartridge (CU) which carries the catalytic beds (C1, C2, C3, ...Cn) .

II   -   An upwards flow (A'), from bottom to top, of the above mentioned feeding gas (GR) outside (A'e) or inside (A'i) the exchangers (EX1, EX2, EX3) set in series along the cartridge axis (CU) and arranged in the central empty zone of the catalytic beds (C1, C2, C3), said gas (A') being thus pre-heated by the reacted gas (D) coming form the various catalytic beds (C1, C2, ...Cn), which runs through said exchangers (EX1, EX2, EX3) in the direction from top to bottom, inside respectively outside said exchangers.

III   -   Fresh exchange gas flows (1, 2, 3, ...n) joining the gas (A') mentioned in paragraph II, after that said gas has run through the first (EX1) and respectively the following ones (second exchanger EX2 in case of three catalytic beds), said exchangers (EX1, EX2, EX3) being, according to paragraph II, arranged in a series and regulating its temperature.

IV - Gas flow (A'') coming out from the last exchanger (EX3) as described at points II and III. from top to bottom and from outside (20) to inside (21) through the many catalytic beds (C1, C2. ...Cn) arranged in series. a minor portion (23) of said gas (A'') running preferably with a mainly axial flow through the upper minor portion of each catalytic bed (C1, C2, ... Cn) the remaining gas major portion (24) running with radial flow through the lower major portion of the catalytic bed.

V - Reacted gas flow (25) coming out of the internal cylindrical wall (P1) of each bed, from bottom to top, into a collector (26) formed by the external side of said cylindrical, perforated, internal wall (Pi), and by the internal side of a cylindrical, non-perforated wall (PP) of smaller diameter. inside which are arranged the exchangers (EX1, EX2, ...EXn).

VI - Central outlet flow (FU) of reacted gas in the shell lower part.

The cartridge (CU) carrying the catalyst, with gas distribution as above described, can now be simply realized in various overlapping parts, each one of them being formed by:

4

1 - One external, cylindrical, closed wall (28) forming the cartridge (CU) with joint elements (S1, S2, S3) for the overlapping tightness of the various parts (C1, C2, C3).

2 - Cylindrical walls (external Pe and internal Pi), perforated along the lower major zone (H') of their height (H), the perforated wall (Pi) having a slightly smaller diameter with respect to the external, cylindrical, non-perforated wall (PP) and forming the outlet collector (26) running through each bed.

3 - Cylindrical, internal, non-perforated diaphragm of far smaller diameter compared to the one of the external, cylindrical, non-perforated wall (PP) and slightly smaller than the one of the internal, cylindrical, perforated wall (Pi), forming with said wall the collector (26) of gas outlet from the catalytic bed.

4 - Closed bottom (FO) between cylindrical, external wall (Pe) and internal, non-perforated diaphragm (PP).

5 - Vertical tube-bundle exchanger (EX1, EX2, EX3) without vertical, cylindrical shell, arranged according to the basket axis, with tube-bundle diameter smaller than the non-perforated wall, as stated in paragraph 3, being said exchanger tightly joined with the exchanger of next bed.

5

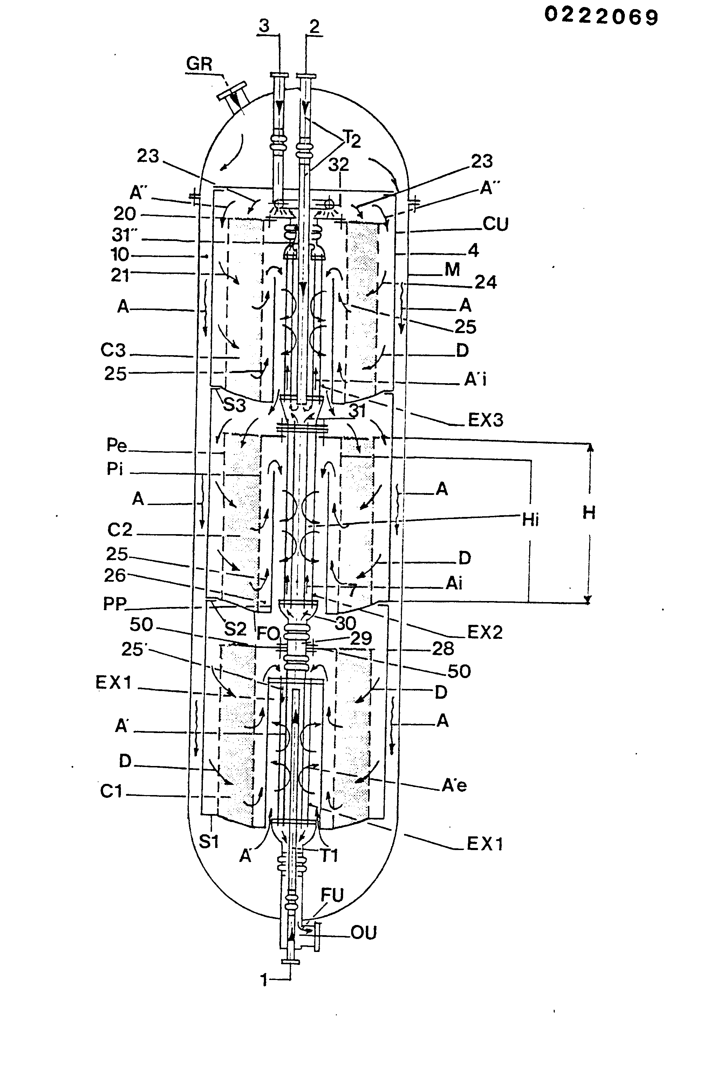

In other words, it has been found that the exothermic reactions of heterogeneous catalytic synthesis are optimized when a critical converter is being used, for instance of the kind shown in the drawing, where:

- the number n of catalytic beds (C1, C2, C3), the number of bundle exchangers ( EX1, EX2, EX3), and the number of adduction means for exchange gas (1, 2, 3) are all identical and for instance, as shown in the drawing, for each bed there is an exchanger and an adduction tube of exchange gas;

- the reaction gas (GR) runs downwards, with a flow (A) from top to bottom between shell (M) and wall (28) of cartridge (CU) and goes up again like a flow (A') from bottom to top, running through the first exchanger (EX1), outside the tube-bundle, with a flow (A'), the tubes of (EX1) being passed inside by the entire amount of reacted gas (25');

- critically the means, or adduction tube (T1) of the first exchange gas flow (1) penetrates from the bottom into the more central zone of the tube-bundle of (EX1), so as to go out in an enlarged mixing body (30) where (A') also arrives, compelled to enter into the tube (29) at top of (EX1);

- the gas (30') in (30) mixture of reation gas (A') and of exchange gas (1) goes now up (EX2) tube-side like flow (Ai), outside said tubes being hit by hot reacted gases (25) gathered in the collector (26):

- said flow (Ai) reaches another closed body (31) where from the top comes the exchange gas flow (2) through the central tube (T2);

- critically the mixture (31') of (31) goes up again, inside the tube-bundle of (EX3) and like flow (31'') it goes into the open space (32) at the cartridge top (CU) where the further exchange flow (3) arrives.

The mixture (32') in (32) is the one coming down again like flow (A'') and making a way into the various catalytic beds (C1, C2, C3);

In the drawing and in the description reference has been made - for simplicity sake - to axial-radial reactors according to previous patents and patent applications of the Applicant (the content of which is to be considered incorporated in this description) but it is obvious that the solution idea, according to the invention is also applicable to other types of converters that differ from the axial-radial one (for instance, simply radial ones).

CLAIMS
------

1. Converters for heterogeneous synthesis and more particularly for ammonia, methanol and higher alcohols catalytic synthesis, which consist of an external shell, of an internal cartridge involving a non-perforated external wall forming an interspace with the shell internal wall, of n catalytic beds, formed by granular catalyst arranged between the closed bottom and cover, both closed, and two cylindrical, concentric, perforated walls, of means for adduction of the reaction gases. of means for extraction of the reacted gases. of means for extraction of the reacted gases. of heat exchangers lined up axially at the center of the catalytic beds. which consist of tube-bundles, and of means for adduction to said tube-bundles of feed gas for heat exchange, characterized by:

- n means of adduction of feed gas for heat exchange, n-1 of which centrally pass through each catalytic bed.

- means to lead the reaction gas flowing from top to bottom in the interspace between shell and cartridge. by making it pass externally to the tube-bundle of at least one lower heat exchanger. which is internally passed by the reacted gases directed to the converter outlet. and by making said reaction gas pass internally to the tube-bundles of the other heat exchangers on its way back to the converter top

- means to mix at the top of each exchanger said reaction gas and said exchange gases.

8

2. Converters, according to claim 1, characterized by the fact that at least one of the two walls of each bed has a non-perforated, minor zone, and that the reaction gas axially crosses the catalytic bed in said non-perforated zone, and radially in its entire remaining zone.

3. Converters, according to claim 2, characterized by the fact that the bed wall with non-perforated minor zone is the internal one, and that it forms an interspace with a non-perforated concentric wall, which is still more internal and extending upwards from the bottom, for an axial extension slightly inferior than the one of the perforated major portion of said internal wall in contact with the catalyst.

4. Converters, according to claim 1, characterized by the fact that maximum n-2 of n-1 adduction means of gas exchange flow into closed spaces at the top on n-2 exchangers, spaces in which occurs the mixing with the reaction gas that first externally and then internally has passed the tube-bundles, and that at least one of said adduction means flows into one open space at the top of the converter.

5. Optimized converters, according to claim 1, characterized by:

 - three catalytic beds, having at least one internal wall with non perforated, minor zone, forming an interspace with a still more internal, non-perforated wall, where reacted gases are collected.

9

- three tube-bundle heat exchangers, centrally fitted to the catalytic beds.

- three adduction tubes of exchange gases, two of them centrally crossing two exchangers and flowing in closed mixture space, and the third adduction tube flowing to the open space on top of the cartridge.

- said elements being coordinated so that the reaction gas passes outside the tube-bundle of the low exchanger and inside the bundles' tube of the other two on upper exchangers.

5. Process for the optimization of the synthesis reaction of ammonia, methanol and alcohols. characterized by the fact that:

- the reaction gas fed from the top downwards. is to go up again and reach the converter top, passing outside the lowest heat exchanger's tubes. is mixed with exchange gas in a closed space at the outlet of said lowest exchanger, is to pass inside the tubes of an intermediate exchanger and of one on upper exchanger, at the outlet of each one being mixed with exchange gas it is then to pass from the top to the bottom with axial-radial flows in three successive catlytic beds and is collected from an outlet at the converter bottom.